# EUROPÄISCHE PATENTSCHRIFT

(11) **EP 0 758 655 B1**
(45) Veröffentlichungstag und Bekanntmachung des Hinweises auf die Patenterteilung: **15.01.2003**
(21) Anmeldenummer: 96112611.7
(22) Anmeldetag: 05.08.1996
(51) Int. Cl.: C07K 14/815, C12P 21/02, C12N 9/00, C12N 1/18, C12N 9/48, A61K 41/00

(54) **Verfahren zur Inaktivierung von Carboxypeptidase Y in hirudinhaltigen Kulturbrühen**
Process for the inactivation of carboxypeptidase Y in hirudin-containing culture broth
Procédé d'inactivation de la carboxypeptidase Y dans les bouillons de culture contenant de l'hirudine

(30) Priorität: 16.08.1995 DE 19529997
(43) Veröffentlichungstag der Anmeldung: 19.02.1997
(73) Patentinhaber: SCHERING AKTIENGESELLSCHAFT, 13342 Berlin (DE)
(72) Erfinder: Crause, Peter, Dr., 63069 Offenbach (DE); Habermann, Paul, Dr., 65817 Eppstein (DE); Möller, Jörg, Dr., 65812 Bad Soden (DE); Ulmer, Wolfgang, Dr., 65817 Eppstein (DE)

(56) Entgegenhaltungen:
- EP-A- 0 168 342
- GB-A- 2 249 096
- ACTA ALIMENTARIA, Bd. 22, Nr. 3, 1993, Seiten 193-209, XP000609841 HALSZ ET AL.: "Proteolytic enzyme activity of S. cerevisiae baker's yeast and S. carlsbergensis brewer's yeast"
- BIOCHEMISTRY, Bd. 13, Nr. 19, 1974, Seiten 3871-3877, XP002019472 KUHN ET AL.: "Isolation and partial characterization of an acid carboxypeptidase from yeast"
- J. BIOL. CHEM., Bd. 266, Nr. 17, 15.Juni 1991, Seiten 10839-10843, XP002019473 CHANG: "Stability of Huridin, a thrombin-specific inhibitor"

## Beschreibung

Die Erfindung betrifft ein Verfahren zur Inaktivierung von Carboxypeptidase Y (CPY) in einer hirudinhaltigen, durch Fermentation einer transformierten Hefe gewonnenen Kulturbrühe.

Bei dem ursprünglich aus dem Blutegel Hirudo medicinalis isolierten Polypeptid Hirudin handelt es sich um einen hochspezifischen Thrombininhibitor mit breitem therapeutischen Potential (F. Markwardt, Biomed. Biochim. Acta 44 (1985) 1007-1013). Die benötigten Mengen können jedoch nur auf gentechnologischem Wege über transformierte Mikroorganismen hergestellt werden. Dabei hat es sich gezeigt, daß die Hefe Saccharomyces cerevisiae als Wirtsorganismus geeignet ist, um korrekt gefaltetes und voll aktives Hirudin zu produzieren (EP A1 168 342, EP A1 200 655).

Die zur gentechnologischen Herstellung von rekombinanten Peptiden und Proteinen besonders bevorzugt eingesetzte Hefe Saccharomyces cerevisiae bildet das Enzym Carboxypeptidase Y (CPY). Dieses Enzym spaltet unspezifisch verschiedene Aminosäuren vom C-Terminus von Proteinen ab und kann deshalb zur Bestimmung der carboxyterminalen Sequenz von Proteinen verwendet werden. Natürlich ist dieses Enzym auch in der Lage, die in der Hefe exprimierten Protein-Wertstoffe, z.B. pharmakologische Wirkstoffe wie etwa Hirudin, während des Herstellverfahrens, insbesondere bei der chromatographischen Reinigung, abzubauen. Dies führt zu Nebenproduktbildungen und Ausbeuteverlusten, die signifikant sein können. Zudem kann es die Stabilität hochgereinigter Produkte herabsetzen, falls es noch in Spuren in der Zubereitung, z.B. in einem Lyophilisat, vorhanden ist.

Eine Lösung des Problems ist die Verwendung von mutierten Hefe-Stämmen, in denen durch verschiedene Methoden die CPY-Aktivität unterdrückt oder eliminiert wird (z.B. EP 390 676, EP 341 215 und GB 2 249 096). Diese Methoden bedingen jedoch eine relativ starke Veränderung des Hefestammes und eventuell Einschränkungen im Wachstumsverhalten und/oder der physiologischen Stabilität der Mikroorganismen.

Eine Inhibierung der CPY gelingt auch mit chemischen Inhibitoren, z.B. mit Phenylmethylsulfonylfluorid (PMSF). Eine solche chemische Inhibierung ist zumindest bei Pharma-Wirkstoffen unerwünscht, da sie zu teilweise nicht detektierbaren partiellen Derivatisierungen führen kann.

Außerdem ist die thermische Instabilität der CPY beschrieben (Kuhn et al.: "Isolation and Partial Characterisation of an Acid Carboxypeptidase from Yeast", Biochemistry, Vol. 13, No. 19, pp 3871-3877, 1974). So führt eine 5-minütige Wärmebehandlung bei 68°C und pH = 7,0 zu einer vollständigen Inaktivierung der CPY; niedrigere Temperaturen führen nur zu partiellen Inaktivierungen.

Das mit der Hefe Saccharomyces cerevisiae exprimierte und ins Kulturmedium sezernierte 7.000 Dalton Protein Hirudin verfügt dagegen über eine bemerkenswerte thermische Stabilität (Jui-Yoa Chang: "Stability of Hirudin, a Thrombin-specific Inhibitor", The Journal of Biological Chemistry, Vol. 266, No. 17, pp 10839-10843, 1991); so werden z.B. nach einer 30-minütigen Behandlung bei 95°C und pH = 8,0 noch 95 % der Ausgangsaktivität wiedergefunden. Andererseits verfügt Hirudin über einen frei liegenden C-Terminus und ist deshalb einem enzymatischen Angriff der CPY weitgehend ungeschützt ausgeliefert.

Demgegenüber ist es Aufgabe der vorliegenden Erfindung, unter Nutzung der entgegengesetzten Eigenschaften von CPY (thermische Instabilität) und Hirudin (thermische Stabilität) ein Verfahren zur Inaktivierung von Carboxypeptidase (CPY) in einer hirudinhaltigen, durch Fermentation einer transformierten Hefe gewonnenen Kulturbrühe bei hohem Hirudinerhalt bereitzustellen.

Eigene Laborexperimente mit hirudinhaltigen Fermentationsbrühen von transformierten Hefen in einem Glaskolben zeigen, daß eine Temperatur von 60°C eine Aktivierung der CPY, offensichtlich aus inaktiven Vorstufen des Enzyms, bewirkt. Dies führt zu einem verstärkten enzymatischen CPY-Angriff auf den freiliegenden C-Terminus des Hirudins und zu C-terminal verkürzten Hirudinen. 80°C andererseits führen bereits zu verstärkter Nebenproduktbildung infolge chemischen Abbaus des Hirudins. Die Erhitzung innerhalb von 10 Minuten auf 70°C mit einer darauffolgenden Inaktivierungszeit von 20 Minuten und anschließender Kühlung auf Raumtemperatur führt zur Inaktivierung der CPY und hoher Hirudin-Wiederfindung.

Ein derart enger Temperatur/Zeit-Korridor zur sicheren Inaktivierung bei hohem Produkterhalt läßt sich nicht ohne weiteres in ein großtechnisches Verfahren umsetzen, außerdem sind die ermittelten Aufheiz- bzw. Abkühlzeiten technisch beispielsweise in einem Rührkessel infolge deren Volumenabhängigkeit sehr schwer realisierbar. Es wurde jetzt gefunden, daß eine kontinuierliche Behandlung in Durchflußwärmetauschern zu einem deutlich verbreiterten sicheren Prozeßbereich führt.

Die vorliegende Erfindung betrifft somit ein Verfahren zur Inaktivierung von Carboxypeptidase Y in einer hirudinhaltigen, durch Fermentation einer transformierten Hefe gewonnenen Kulturbrühe, welches sich dadurch auszeichnet, daß die Kulturbrühe unter Durchfluß einer rohrförmigen Apparatur, welche eine Aufheiz-, eine Verweilzeit- und eine Abkühlstrecke aufweist, kontinuierlich auf eine Temperatur von 80 bis 100°C, vorzugsweise 85 bis 95°C, besonders bevorzugt auf eine Temperatur von 85°C erhitzt wird.

Im folgenden wird die vorliegende Erfindung detailliert beschrieben, insbesondere in ihren bevorzugten Ausführungsformen.

Die Apparatur zur Inaktivierung der CPY besteht in der einfachsten Ausführung aus einem, gegebenenfalls mit statischen Mischern ausgestatteten, beheizbaren Rohr, beispielsweise einem Durchflußwärmetauscher, als Aufheizstrecke, in welcher die Kulturbrühe auf den bevorzugten Temperaturbereich gebracht wird und einem isolierten Rohr als Verweilzeit- oder Inaktivierungsstrecke, in welcher die Kulturbrühe auf dem bevorzugten Temperaturbereich gehalten wird, sowie einem gekühlten Rohr , beispielsweise einem Durchflußwärmetauscher, als Abkühlstrecke, in welcher die Kulturbrühe auf Raumtemperatur abgekühlt wird.

Bei dem Verfahren gemäß der vorliegenden Erfindung beträgt die mittlere Verweilzeit der Kulturbrühe in der Aufheizstrecke der Apparatur vorzugsweise 40 bis 60 Sekunden.

Die mittlere Verweilzeit kann durch die Apparateauslegung und den Durchfluß eingestellt werden. Die Apparatur wird zunächst mit demineralisiertem, filtriertem Wasser angefahren. Sobald am Ende der Aufheizstrecke der bevorzugte Temperaturbereich erreicht ist, wird der Zufluß von Wasser auf die Kulturbrühe umgestellt.

Nach Inaktivierung der Kulturbrühe wird die Apparatur wiederum unter Betriebsbedingungen mit demineralisierten, filtriertem Wasser leer gefahren, um Produktverluste in der Anlage zu minimieren. Erst danach wird die Beheizung abgestellt.

Eine Temperatur von 70°C führt zu einem starken Aktivitätsanstieg der CPY verbunden mit einem verstärkten Hirudinabbau. Bei 75°C wird noch eine Restaktivität an CPY von ca. 2 % des ursprünglichen Wertes und bei 80°C von ca. 0,5 % beobachtet. Temperaturen zwischen 85°C und 95°C reduzieren die Ausgangsaktivität auf ca. 0,1 % und bringen eine Produktausbeute von 95 %; teilweise werden sogar quantitative Ausbeuten erzielt. Die so behandelten Produktlösungen sind stabil und zeigen keinen weiteren enzymatischen Abbau. Erst bei Temperaturen > 100°C führt ein chemischer Abbau zu großen Produktverlusten.

Da die eingesetzte Kulturbrühe nur vorgereinigt ist, sind neben dem Produkt noch diverse andere Proteine/Nebenprodukte sowie Nukleinsäuren und andere organische und anorganische Bestandteile in der zu inaktivierenden Lösung vorhanden. Um Ablagerungen und Anbackungen auf den Wärmetauscheroberflächen, die den Wärmetransport behindern und die Temperatur erniedrigen können, zu vermeiden, besteht die Aufheizstrecke der Apparatur bei dem Verfahren gemäß der vorliegenden Erfindung vorzugsweise aus solchen Wärmetauschern, bei denen die innere Oberfläche kontinuierlich mechanisch gereinigt wird.

Besonders bevorzugt werden sogenannte Schabewärmetauscher eingesetzt, die kommerziell erhältlich sind und großtechnische Anwendung beispielsweise zur Pasteurisierung von Fruchtsäften oder Molkereiprodukten finden. Bei diesen Geräten laufen aus Kunstharz hergestellte Schaber auf einer Welle innerhalb des Erhitzerrohres und werden durch die Fliehkraft an die Rohrwandungen gedrückt, wodurch Anbackungen kontinuierlich abgetragen werden. Jedoch sind sämtliche andere Vorrichtungen zur Verhinderung von Ablagerungen und Anbackungen auf den inneren Wärmetauscheroberflächen für das Verfahren gemäß der vorliegenden Erfindung einsetzbar.

Bei dem erfindungsgemäßen Verfahren werden hirudinhaltige Kulturbrühen aus transformierten Hefen, besonders bevorzugt aus Saccharomyces cerevisiae eingesetzt.

Der pH-Wert der Kulturbrühe beträgt vorzugsweise 6,2 bis 6,5.

Wahlweise kann die Erhitzung der Kulturbrühe in der Aufheizstrecke der Apparatur mittels Mikrowellen erfolgen.

Die vorliegende Erfindung findet Anwendung für die Inaktivierung von Carboxypeptidase Y (CPY) in Kulturbrühen, die ein rekombinantes Hirudin enthalten, insbesondere solches Hirudin, welches in Saccharomyces cerevisiae zur Expression gebracht wird.

Unter Hirudin sind peptidartige Thrombininhibitoren mit einer spezifischen Aktivität von mindestens 10000 AT-U/mg zu verstehen, die von den bekannten Isohirudinen der Spezies Hirudo medicinalis abgeleitet sind und wesentliche Strukturmerkmale dieser, insbesondere die charakterisistische Verknüpfung der drei Disulfidbrücken (J. Dodt et al., Biol. Chem. Hoppe-Seyler 366 (1985) 379-385), aufweisen (vgl. z.B. EP A1 158 564, EP A1 168 342, DE 34 45 517, EP A2 193 175, EP A1 200 655, EP A1 158 986, EP A1 209 061, DE 33 42 199, EP A1 171 024).

Insbesondere wird darunter ein Hirudin verstanden, wie es in der EP A1 171 024, der EP A1 158 986 und der EP A1 209 061 beschrieben ist.

Das erfindungsgemäße Verfahren wird besonders bevorzugt zur Inaktivierung von CPY in einer Kulturbrühe eingesetzt, die ein Hirudinderivat mit der in der EP 0 324 712 offenbarten Aminosäuresequenz enthält. ([Leu¹, Thr²]-63-Desulfohirudin).

Durch Anwendung dieser hier offenbarten Inaktivierungsmethode wird die Aufarbeitung und Hochreinigung von hirudinhaltigen Lösungen überhaupt erst ermöglicht, ohne durch Mutation und/oder gentechnologische Methoden den Produktionsstamm zu verändern oder die Lösungen durch die Einwirkung chemischer Inhibitoren zu verunreinigen.

### Beispiele

Die nachfolgenden Beispiele betreffen durch Fermentation von transformierten Hefen gewonnene Kulturbrühen, welche das Hirudinderivat mit der in der EP 0 324 712 offenbarten Aminosäuresequenz ([Leu¹, Thr²]-63-Desulfohirudin) enthalten. Die gleichen vorteilhaften Ergebnisse lassen sich jedoch auch mit anderen, insbesondere den eingangs erwähnten Hirudinen erzielen.

### Allgemeine Vorschrift:

Die zu inaktivierende Lösung, die bedingt durch Ausfällungen trüb und auf Temperaturen T ≤ 10°C gekühlt ist, wird mit Essigsäure oder Natronlauge auf einen pH-Wert von pH = 6,4 ± 0,1 eingestellt. Anschließend wird die Apparatur zur Hitzeinaktivierung, bestehend aus einem Schabewärmetauscher der Fa. APV Crepaco, Typ 1HO-648S/CP 508 als Erhitzer, einer Verweilzeitstrecke und einem Kühler mit demineralisiertem, filtriertem Wasser (purified water) auf Betriebstemperatur angefahren. Die mittleren Verweilzeiten in den einzelnen Abschnitten des Wärmetauschers werden durch den gewählten Durchsatz eingestellt und mit Hilfe von Tracer-Experimenten (Puls-Zugabe eines Fluoreszenzfarbstoffes und Detektion mit einem Fluoreszenzsensor) bestimmt. Sobald am Ende des Erhitzers die gewünschte Temperatur (± 1 °C) erreicht ist, wird der Zufluß von Wasser auf Produktlösung umgestellt. Nach Inaktivierung der Lösung wird die Apparatur wiederum unter Betriebsbedingungen mit demineralisiertem, filtriertem Wasser (purified water) leer gefahren, um Produktverluste in der Anlage zu minimieren; erst danach wird die Beheizung abgestellt. Zur Abtrennung von bei der Hitzebehandlung ausfallenden Präzipitats wird die Suspension filtriert.

Die CPY-Konzentrationen werden mittels des nachfolgend beschriebenen ELISA bestimmt.

Der CPY-ELISA wird im Prinzip nach dem Fachmann bekannten Verfahren durchgeführt. (Lit.: P. Tijssen, Practice and Theory of Enzyme Immunoassays, in Laboratory Techniques in Biochemistry and Molecular Biology (Burdon, R. H., van Knippenberg, P. H., Eds.), Vol. 15, Elsevier 1985).

Folgende spezielle Reagenzien werden benutzt:

| | | |
|---|---|---|
| Beschichtungspuffer | NaH₂PO₄*2 H₂O | 0,315 g |
| | Na₂HPO₄*2 H₂O | 1,2 g |
| | Natriumazid | 0,5 g |

auf 1 Liter mit Wasser aufgefüllt.

| | | |
|---|---|---|
| Blockierpuffer | 1 g Rinderserumalbumin (BSA)/1 I PBS (Phosphate Buffered Saline) | |
| MSTB-Puffer | MOPS (Morpholinopropansulfonsäure) | 10,45 g |
| | SDS | 1,0 g |
| | Triton X-100 | 1,0 g |
| | BSA | 12,5 g |

auf 500 ml mit Wasser aufgefüllt, mit NaOH auf pH 7.4 eingestellt.

| | |
|---|---|
| Waschpuffer | 0,5 g Tween 20/1 I PBS |
| Konjugatpuffer | Mikrobiol für Enzygnost®, Behringwerke Marburg, Best. Nr. OUWW |
| TMB (Tetramethylbenzidin) | Combipack-Zusatzreagenzien für Enzygnost®/TMB, Behringwerke Marburg, Best. Nr. OUVP 10/11 |
| CPY | Carboxypeptidase (Yeast), 135 U/mg, z. B. Serva Heidelberg, Best Nr. 16137 |
| Beschichtungsantikörper | Schaf anti-CPY, hergestellt nach dem Fachmann bekannten Verfahren |
| Nachweisantikörper | Kaninchen-anti-CPY, hergestellt nach dem Fachmann bekannten Verfahren, markiert mit Peroxidase. |

### Durchführung:

Mikrotest-Platten werden mit Beschichtungsantikörper (5 µg/ml in Beschichtungspuffer, 100 µl/Bohrung) gefüllt und mindestens 7 Tage lang bei 10 °C inkubiert. Anschließend werden die Beschichtungslösung entfernt und 200 µl Biockierpuffer/Bohrung zugegeben und die Platten bei 37 °C für 90 min. inkubiert. Danach werden die CPY-Proben aufgetragen. Bei jedem Versuch wird eine Standard-Reihe mitgeführt (1 µg/ml - 0,002 ng/ml CPY in MSTB-Puffer, 1 : 3 Verdünnungen). In Abhängigkeit von der erwarteten Konzentration an CPY werden die unbekannten Proben in MSTB-Puffer vorverdünnt. Alle Proben werden nach Entfernen des Blockierpuffers mit 100 µl/Bohrung auf die Platten gegeben. Anschließend werden die Platten bei 37 °C 90 min. inkubiert. Danach werden die Platten drei mal mit Waschpuffer gewaschen. Der Nachweisantikörper wird in geeigneter Weise, z. B. 1 : 10.000, in Mikrobiol-Puffer + 1 % normales Kaninchenserum verdünnt und in jede Bohrung 100 µl davon pipettiert. Anschließend werden die Platten 90 min. bei 37 °C inkubiert und danach drei mal mit Waschpuffer gewaschen. Der Nachweis der gebundenen Peroxidase erfolgt mit den Reagenzien aus dem Combipack Zusatzreagenzien für Enzygnost®/TMB: das TMB-Chromogen wird 1 : 20 in Puffer/Substrat verdünnt und auf Raumtemperatur erwärmt. Davon werden je 100 µl in jede Bohrung der Platten gegeben. Die Platten werden dann 30 min. bei Raumtemperatur und unter Schutz vor Lichteinwirkung inkubiert. Danach wird die Reaktion durch Zugabe von 100 µl 0,5 M H₂SO₄ gestoppt und die Farbentwicklung in einem Plattenphotometer bei 450 nm gemessen. Die Berechnung der CPY-Konzentration in den unbekannten Proben erfolgt unter Zugrundelegung der Standardreihe mit einem geeigneten Computerprogramm wie z. B. KinetiCalc® von Tecnomara, Fernwald.

### Beispiel 1:

Aufheizung auf Inaktivierungstemperatur, Inaktivierung und Abkühlung auf Raumtemperatur sind durch die Apparateauslegung (Durchsatz 400 l/h ± 20 l/h) jeweils auf bis zu 60 Sekunden (mittlere Verweilzeit) eingestellt worden. Mit folgenden mittleren Verweilzeiten sind die nachstehend aufgeführten Ergebnisse erzielt worden:
Aufheizstrecke: 50 sec
Inaktivierungsstrecke: 28 sec
Abkühlstrecke: 12 sec

**Tabelle 1**

| Inaktivierung der CPY bei verschiedenen Temperaturen | |
|---|---|
| Temperatur | CPY [ng/ml] |
| 75°C | 16,3 |
| 80°C | 4,1 |
| 85°C | 1,1 |
| 90°C | 0,9 |
| 95°C | 1,0 |
| Ausgangsprobe | 760 |

Die CPY-Konzentrationen der Proben werden mittels ELISA bestimmt. Die Nachweisgrenze dieses Tests liegt bei 0,5 ng/ml.

### Beispiel 2:

Durch Variation des Durchsatzes wurden in der in der allgemeinen Vorschrift beschriebenen Apparatur verschiedene Verweilzeiten in der Aufheizstrecke eingestellt. Die Temperatur am Ende der Aufheizstrecke und in der Inaktivierungsstrecke betrug 85°C. In Tabelle 2 sind die nach Behandlung der Kulturbrühe mittels ELISA bestimmten CPY-Konzentrationen gegen die mittleren Verweilzeiten in der Aufheizstrecke aufgetragen.

**Tabelle 2**

| CPY-Konzentration nach Inaktivierung bei 85°C (variierte mittlere Verweilzeiten bzw. Aufheizzeiten) | | |
|---|---|---|
| Durchsatz [l/h] | mittlere Verweilzeit [s] | CPY [ng/ml] |
| 340 | 59 | 1,13 |
| 400 | 50 | 1,2 |
| 500 | 40 | 1,16 |

### Beispiel 3:

Nach dem in der allgemeinen Vorschrift beschriebenen Verfahren wurden hirudinhaltige Kulturbrühen bei verschiedenen Temperaturen behandelt, und der Hirudingehalt nach Sterilfiltration (0,2 µm Filter) jeweils sofort und nach 8 Tagen Aufbewahrung der Proben bei 2 bis 8°C mittels HPLC (Bedingungen: stationäre Phase: LiChrospher® 300 RP-18, 10 µm (Merck); mobile Phase A: Acetonitril 1600 ml/bidest. Wasser 400 ml/ Trifluoressigsäure 2 ml; mobile Phase B: bidest. Wasser 2000 mi/Trifluoressigsäure 2 ml; Gradient: A/B = 20 : 80 % - 80 : 20 %; Fluß: 1,5 ml/min; T = 45 °C) bestimmt. In Tabelle 3 sind die Ergebnisse dargestellt.

**Tabelle 3:**

| Inaktivierungstemperatur [°C] | Gehalt an [Leu¹, Thr²]-63-Desulfohirudin [%] | |
|---|---|---|
| Ausgangslösung | Testanfang 100 | Testende (nach 8 Tagen) 50 |
| 108 | 68 | 68 |
| 100 | 93 | 93 |
| 90 | 92 | 92 |
| 85 | 99 | 97 |
| 80 | 92 | 89 |
| 70 | 81 | 19 |

## Patentansprüche

1. Verfahren zur Inaktivierung von Carboxypeptidase Y in einer hirudinhaltigen, durch Fermentation einer transformierten Hefe gewonnenen Kulturbrühe, **dadurch gekennzeichnet, dass** die Kulturbrühe unter Durchfluß einer rohrförmigen Apparatur, aufweisend eine Aufheiz-, eine Verweilzeit- und eine Abkühistrecke, bei einem pH-Wert von 6,2 bis 6,5 kontinuierlich auf eine Temperatur von 80° bis 100°C erhitzt wird.

2. Verfahren nach Anspruch 1, **dadurch gekennzeichnet, dass** die Temperatur 85° bis 95°C beträgt.

3. Verfahren nach Anspruch 1, **dadurch gekennzeichnet, dass** die Temperatur 85°C beträgt.

4. Verfahren nach einem der Ansprüche 1 bis 3, **dadurch gekennzeichnet, dass** die mittlere Verweilzeit der Kulturbrühe in der Aufheizstrecke der Apparatur 40 bis 60 Sekunden beträgt.

5. Verfahren nach einem der Ansprüche 1 bis 4, **dadurch gekennzeichnet, dass** die Aufheizstrecke der Apparatur aus einem Durchflußwärmetauscher besteht, dessen innere Oberfläche kontinuierlich mechanisch gereinigt wird.

6. Verfahren nach einem der Ansprüche 1 bis 5, **dadurch gekennzeichnet, dass** die transformierte Hefe Saccharomyces cerevisiae ist.

7. Verfahren nach Anspruch 1, **dadurch gekennzeichnet, dass** die Kulturbrühe durch Einwirkung von Mikrowellen erhitzt wird.

## Claims

1. A process for inactivating carboxypeptidase Y in a hirudin-containing culture broth produced by fermenting a transformed yeast, which comprises continuously heating the culture broth, flowing through a tubular apparatus which has a heating-up section, a holding section and a cooling section, to a temperature of 80 to 100°C at a pH of 6.2 to 6.5.

2. The process as claimed in claim 1, wherein the temperature is 85 to 95°C.

3. The process as claimed in claim 1, wherein the temperature is 85°C.

4. The process as claimed in one of claims 1 to 3, wherein the mean residence time of the culture broth in the heating-up section of the apparatus is 40 to 60 seconds.

5. The process as claimed in one of claims 1 to 4, wherein the heating-up section of the apparatus comprises a flow-through heat exchanger whose inner surface is continuously mechanically cleaned.

6. The process as claimed in one of claims 1 to 5, wherein the transformed yeast is Saccharomyces cerevisiae.

7. The process as claimed in claim 1, wherein the culture broth is heated by the action of microwaves.

## Revendications

1. Procédé d'inactivation de carboxypeptidase Y dans un bouillon de culture contenant de l'hirudine, obtenue par fermentation d'une levure transformée, **caractérisé en ce que** le bouillon de culture est chauffé de façon continue à une température de 80° à 100°C, à une valeur de pH de 6,2 à 6,5 lors d'un passage à travers un appareillage tubulaire présentant une section de chauffage, une section de séjour et une section de refroidissement.

2. Procédé selon la revendication 1, **caractérisé en ce que** la température s'élève de 85° à 95°C.

3. Procédé selon la revendication 1, **caractérisé en ce que** la température s'élève à 85°C.

4. Procédé selon l'une quelconque des revendications 1 à 3, **caractérisé en ce que** le temps de séjour moyen du bouillon de culture dans la section de chauffage de l'appareillage s'élève de 40 à 60 secondes.

5. Procédé selon l'une quelconque des revendications 1 à 4, **caractérisé en ce que** la section de chauffage de l'appareillage est constituée par un échangeur de chaleur à passage continu dont la surface interne est nettoyée mécaniquement en permanence.

6. Procédé selon l'une quelconque des revendications 1 à 5, **caractérisé en ce que** la levure transformée est Saccharomyces cerevisiae.

7. Procédé selon la revendication 1, **caractérisé en ce que** le bouillon de culture est chauffé par action de micro-ondes.
